(19) Europäisches Patentamt
European Patent Office
Office européen des brevets

(11) Veröffentlichungsnummer: **0 257 420 B1**

# (12) EUROPÄISCHE PATENTSCHRIFT

(45) Veröffentlichungstag der Patentschrift: **16.05.90**

(51) Int. Cl.⁵: **C07C 265/12**, C07C 263/00, C07C 201/06, C08G 18/77

(21) Anmeldenummer: **87111535.8**

(22) Anmeldetag: **10.08.87**

## (54) Ausgewählte Diisocyanate, Verfahren zu deren Herstellung und deren Verwendung als Aufbaukomponente bei der Herstellung von Polyurethankunststoffen.

(30) Priorität: **21.08.86 DE 3628316**

(43) Veröffentlichungstag der Anmeldung: **02.03.88 Patentblatt 88/9**

(45) Bekanntmachung des Hinweises auf die Patenterteilung: **16.05.90 Patentblatt 90/20**

(84) Benannte Vertragsstaaten: **BE DE ES FR GB IT NL SE**

(56) Entgegenhaltungen:
**EP-A- 0 183 115**
**US-A- 4 328 166**

**Chemical Abstracts, Band 49, Nr.1, 10 Januar 1955, Columbus, Ohio, USA F.H. Mcmillan "Diaryloxyalkane derivatives. Some miscellaneous diphenoxypropanes" Zusammenfassung-Nr. 214a-d**

(73) Patentinhaber: **BAYER AG, D-5090 Leverkusen 1 Bayerwerk(DE)**

(72) Erfinder: **Sanders, Josef, Dr., Wolfskaul 6, D-5000 Köln 80(DE)**

EP 0 257 420 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Diisocyanate mit Bis(isocyanatophenoxy)-alkan-Struktur, ein Verfahren zur Herstellung dieser Diisocyanate und deren Verwendung als Aufbaukomponente bei der Herstellung von Polyurethankunststoffen nach dem Isocyanat-Polyadditionsverfahren.

In der DE-OS 3 442 689 werden bereits bestimmte Bis-(4-isocyanatophenoxy)-alkane beschrieben, welche bezüglich ihrer Eignung zur Herstellung von hochwertigen Polyurethanelastomeren dem 1, 5-Diisocyanatonaphthalin vergleichbar sind. Konkret werden jedoch nur Diisocyanate beschrieben, die unverzweigte Alkylenreste zwischen den beiden Ethersauerstoffen aufweisen. Diese bekannten Diisocyanate weisen jedoch sowohl bezüglich ihrer Eigenschaften als auch bezüglich ihrer Herstellbarkeit, d.h. bezüglich der Herstellbarkeit der ihnen zugrundeliegenden Vorprodukte gewisse Nachteile auf. So liegen ihre Schmelzpunkte mit 88 bis 98°C relativ hoch und auch ihre Löslichkeit in unpolaren organischen Lösungsmitteln ist unbefriedigend. Infolgedessen ist ihre Verarbeitung in der Schmelze und in Lösung häufig problematisch und vergleichsweise unwirtschaftlich. Auch die Herstellung der ihnen zugrundeliegenden Dinitroverbindungen nach dem bisherigen Stand der Technik ist unwirtschaftlich. Die Kondensation von Alkali-4-nitrophenolat mit Alkandihalogeniden ergibt nur bei Einsatz von teuren Alkandibromiden akzeptable Ausbeuten. Ein Teil dieser teuren Dibromide wird zudem in einer Nebenreaktion unter Eliminierung von Bromwasserstoff irreversibel verbraucht. Diese Nebenreaktion kann bei Einsatz von verzweigten Alkandihalogeniden zur Hauptreaktion werden. Sterisch gehinderte Alkandihalogenide, z.B. 1, 3-Dihalogen-2,2-dialkylpropane können nach diesen Verfahren überhaupt nicht zu den entsprechenden Bis(nitrophenoxy)-alkanen umgesetzt werden.

Die DE-OS 3 442 689 erwähnt zwar als weitere Methode zur Herstellung der Dinitroverbindungen die Umsetzung von 4-Nitrochlorbenzol mit Alkandiolen oder mit (4-Nitrophenoxy)alkanolen in Gegenwart von Basen, jedoch ohne jeglichen Hinweis auf die Art der einzusetzenden Base bzw. auf gegebenenfalls mitzuverwendende Lösungsmittel. Der bei der Lektüre dieser Offenbarung nächstliegende Gedanke, als Basen wäßrige Natronlauge zu verwenden führt indessen auch bei Verwendung von inerten Lösungsmitteln wie Chlorbenzol zu unbefriedigenden Ausbeuten an den entsprechenden Dinitroverbindungen. Eine derartige Arbeitsweise ist im übrigen für die Herstellung von analogen Dinitroverbindungen mit verzweigtem Alkanrest völlig ungeeignet. Auch der Gedanke als "Base " Alkalimetalle einzusetzen, führt nicht zum gewünschten Ergebnis da bereits im Falle der Verwendung von einwertigen Alkoholen Nebenreaktionen in beträchtlichem Umfang auftreten (vgl. hierzu: The Chemistry of the Ether Linkage, Herausgeber S. Patai, Interscience Publishers, 1967, S. 450; Houben Weyl, Bd. VI/3 S. 77).

Auch die in der DE-OS 2 634 419 beschriebene phasentransferkatalysierte Umsetzung von Nitrochlorbenzolen mit organischen Hydroxyverbindungen in wäßrig/organischen Zweiphasensystemen führt bei Einsatz von 2- bzw. 4-Nitrochlorbenzol und Alkandiolen nur in Spuren zu den gewünschten Bisnitrophenoxyalkanen. Als Hauptprodukte erhält man statt dessen Dichlorazoxybenzole sowie die entsprechenden Nitrophenoxyalkanole,

Es war daher die der Erfindung zugrundeliegende Aufgabe, Diisocyanate zur Verfügung zu stellen, die die oben geschilderten Nachteile bezüglich ihrer Eigenschaften nicht aufweisen und die einfach und in guten Ausbeuten herstellbar sind.

Gegenstand der Erfindung sind somit Diisocyanate der allgemeinen Formel

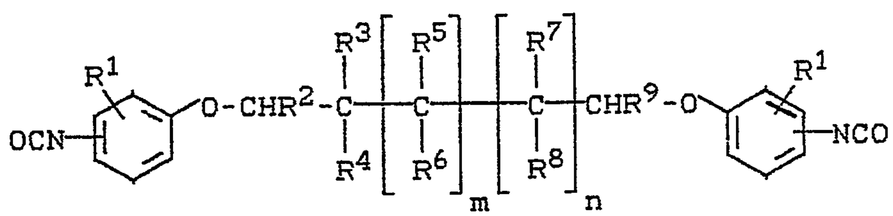

für welche
$R^1$ für Wasserstoff oder eine Methylgruppe steht und
$R^2$, $R^3$, $R^5$, $R_6$, $R^7$, $R^8$ und $R^9$ für Wasserstoff oder Alkylreste mit 1 bis 4 Kohlenstoffatomen stehen,
$R^4$ für einen Alkylrest mit 1 bis 4 Kohlenstoffatomen steht, und
m und n jeweils für 0 oder 1 stehen.

Besonders bevorzugt sind Diisocyanate der obigen Formel für welche
$R^1$ für Wasserstoff steht,
$R^2$, $R^5$, $R^6$, $R^7$ und $R^9$ für Wasserstoff stehen,
$R^3$ und $R^4$ für Methylgruppen stehen,
$R^8$ für Wasserstoff oder eine Methylgruppe steht und
m und n jeweils für 0 oder 1 stehen,
und in welchen die Diisocyanatgruppen ortho- oder paraständig zu dem mit dem aromatischen Ring verknüpften Sauerstoffatom angeordnet sind.

Ein weiterer Gegenstand der Erfindung ist ein Verfahren zur Herstellung der erfindungsgemäßen Diisocyanate durch

a) Umsetzung von (i) ein- oder zweiwertigen Alkoholen der Formeln

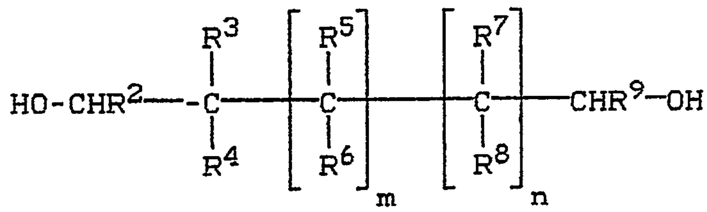

oder

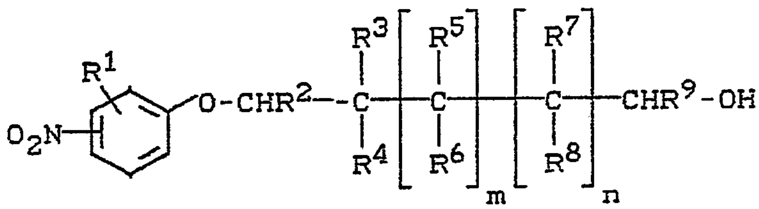

mit (ii) Verbindungen der allgemeinen Formel

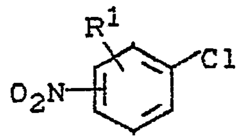

zu den entsprechenden Dinitroverbindungen wobei in diesen Formeln $R^1$ für Wasserstoff oder eine Methylgruppe steht,
$R^2$, $R^3$, $R_5$, $R^6$, $R^7$, $R^8$ und $R^9$ für Wasserstoff oder Alkylreste mit 1 bis 4 Kohlenstoffatomen stehen,
$R^4$ für einen Alkylrest mit 1 bis 4 Kohlenstoffatomen steht und
m und n jeweils für 0 oder 1 stehen,

b) an sich bekannte Hydrierung der zuletzt genannten Dinitroverbindungen zu den entsprechenden diprimären Diaminen und

c) an sich bekannte Phosgenierung der gemäß b) erhaltenen Diaminoverbindungen,

wobei man die Umsetzung a) in Gegenwart von gepulvertem Natrium- und/oder Kaliumhydroxid in einer zur Neutralisation des abgespalteten Chlorwasserstoffs mindestens ausreichenden Menge und in Gegenwart eines stark polaren, aprotischen Lösungsmittels durchführt.

Besonders bevorzugt ist die Herstellung der erfindungsgemäßen Diisocyanate durch

a) Umsetzung von (i) ein- oder zweiwertigen Alkoholen der Formeln

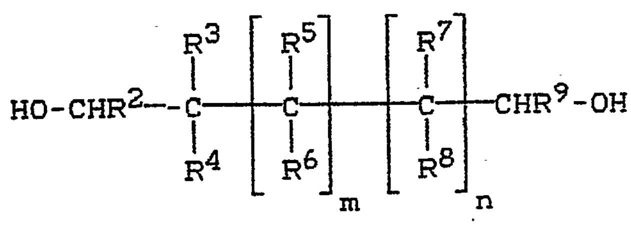

oder

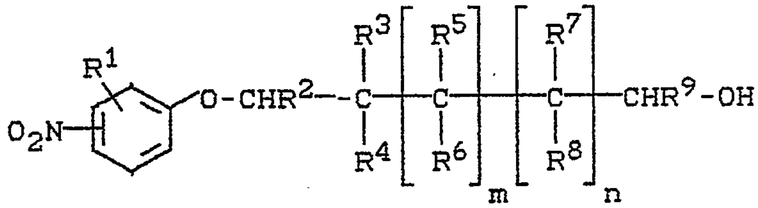

mit
(ii) Verbindungen der allgemeinen Formel

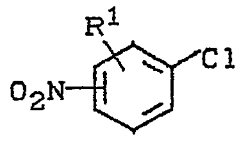

zu den entsprechenden Dinitroverbindungen wobei in diesen Formeln $R^1$ für Wasserstoff steht,
$R^2$, $R^5$, $R^6$, $R^7$ und $R^9$ für Wasserstoff stehen,
$R^3$ und $R^4$ für Methylgruppen stehen, $R^8$ für Wasserstoff oder eine Methylgruppe steht und m und n jeweils für 0 oder 1 stehen.

b) an sich bekannte Hydrierung der zuletzt genannten Dinitroverbindungen zu den entsprechenden diprimären Diaminen und

c) an sich bekannte Phosgenierung der gemäß b) erhaltenen Diaminoverbindungen, wobei man die Umsetzung a) in Gegenwart von gepulvertem Natrium- und/oder Kaliumhydroxid in einer zur Neutralisation des abgespaltenen Chlorwasserstoffs mindestens ausreichenden Menge und in Gegenwart eines stark polaren, aprotischen Lösungsmittels durchführt.

Gegenstand der Erfindung ist schließlich auch die Verwendung der erfindungsgemäßen Diisocyanate als Aufbaukomponente bei der Herstellung von Polyurethankunststoffen nach dem Isocyanat-Polyadditionsverfahren.

Ausgangsmaterialien für das erfindungsgemäße Verfahren sind (i) ein- oder zweiwertige Alkohole und (ii) gegebenenfalls methylsubstituierte Nitrochlorbenzole,

Geeignete alkoholische Aufbaukomponenten (i) sind Diole wie 2-Methyl-1,3-dihydroxypropan, 3-Methyl-1,5-dihydroxypentan oder 2-Ethyl-1,3-dihydroxyhexan.

Zu den bevorzugten Alkandiolen gehören beispielsweise 2,2-Dimethyl-1,3-dihydroxypropan, 2-Methyl-2-propyl-1,3-dihydroxypropan, 2,2-Diethyl-1,3-dihydroxypropan, 2,2,4-Trimethyl-1,3-dihydroxypentan oder 2,2, 4-Trimethyl-1,6-dihydroxyhexan.

Weitere alkoholische Ausgangsmaterialien (i) sind einwertige Alkohole.

Zu den erfindungsgemäß in Betracht kommenden Ausgangsmaterialien (ii) gehören gegebenenfalls methylsubstituierte Nitrochlorbenzole der allgemeinen Formel

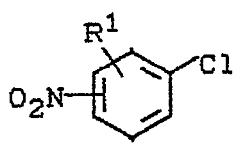

für welche
$R^1$ für Wasserstoff oder eine Methylgruppe, vorzugsweise für Wasserstoff steht,
und in welchen die Chlor- und Nitrosubstituenten vorzugsweise ortho- oder paraständig zueinander angeordnet sind, In Betracht kommen beispielsweise 2-Nitrochlorbenzol, 4-Nitrochlorbenzol, 1-Methyl-2-nitro-3-chlorbenzol, 1-Methyl-4-nitro-5-chlorbenzol oder 1-Methyl-2-nitro-6-chlorbenzol. Besonders bevorzugte Ausgangsmaterialien (ii) sind 2-Nitrochlorbenzol oder 4-Nitrochlorbenzol.

Bei der Durchführung der Stufe a) des erfindungsgemäßen Verfahrens können die Ausgangsmaterialien (ii), bezogen auf die Komponente (i) sowohl in stöchiometrischer Menge als auch im Über- oder Unterschuß zum Einsatz gelangen. Vorzugsweise wird die Menge der Komponente (ii) so bemessen, daß auf jedes Mol an Hydroxylgruppen der Komponente (i) 1 bis 1, 5 Mol der Komponente (ii) vorliegt.

Die Stufe a) des erfindungsgemäßen Verfahrens wird in Gegenwart von gepulvertem Alkalihydroxid, d.h. von gepulvertem Natrium- und/oder Kaliumhydroxid, vorzugsweise gepulvertem Natriumhydroxid durchgeführt. Die mittlere Teilchengröße der gepulverten Alkalihydroxide liegt hierbei im allgemeinen bei 1 bis 25 µm. Derartige gepulverte Natriumhydroxide sind beispielsweise von der Firma Reininghaus-Chemie, D-4300 Essen, im Handel erhältlich.

Vorzugsweise wird die Menge der Alkalihydroxide zumindest so bemessen, daß sie zur Neutralisation des abgespaltenen Chlorwasserstoffs ausreicht. Besonders bevorzugt ist die Verwendung der Alkalihydroxide in einer solchen Menge, daß auf jedes Mol an Hydroxylgruppen der Komponente (i) 1, 3 bis 2 Mol an Alkalihydroxid zur Verfügung steht.

Die Stufe a) des erfindungsgemäßen Verfahren wird in Gegenwart von polaren, aprotischen Lösungsmitteln durchgeführt. Bei diesen Lösungsmitteln handelt es sich vorzugsweise um solche, die ein Dipolmoment von mindestens 2,0, insbesondere von mindestens 3,0 Debye aufweisen. In Betracht kommen beispielsweise Aceton, Acetonitril, Dimethylformamid, Dimethylacetamid, Dimethylsulfoxid, Tetramethylensulfon, Furfurol, Nitromethan, Nitropropan, Nitrobenzol, N-Methylpyrroliden, Hexamethylenphosphorsäuretriamid, Tetramethylharnstoff, Trimethylharnstoff, Ethylenglykol-diethylether oder beliebige Gemische derartiger Lösungsmittel. Vorzugsweise wird Dimethylsulfoxid oder Dimethylformamid verwendet. Die Menge des Lösungsmittels wird hierbei im allgemeinen so bemessen, daß sie einerseits ausreicht, um die Ausgangsmaterialien (i) und (ii) klar zu lösen und andererseits gewährleistet ist, daß die meist festen Dinitroverbindungen nach Beendigung der Kondensationsreaktion beim Abkühlen des Reaktionsgemischs auf Raumtemperatur zum überwiegenden Teil ausfallen und durch einfaches Filtrieren isoliert werden können. Höhere Lösungsmittelmengen sind zwar möglich, aber unwirtschaftlich. In der Praxis bedeutet dies, daß die Lösungsmittel im allgemeinen in einer Menge von 100 bis 300 Gew.-Teilen Lösungsmittel pro 100 Gew.-Teilen des Gemischs aus den Komponenten (i) und (ii) zum Einsatz gelangen.

Zur Durchführung der Stufe a) des erfindungsgemäßen Verfahrens kann man beispielsweise so vorgehen, daß man die Ausgangsmaterialien (i) und (ii) im gewählten Lösungsmittel vorlegt und das feste, pulverförmige Alkalihydroxid unter Rühren, ggf. unter Kühlung portionsweise oder kontinuierlich zugibt. Die Umsetzung wird im allgemeinen innerhalb des Temperaturbereichs von 10 bis 100°C, vorzugsweise von 25 bis 60°C durchgeführt.

Nach Abklingen der exothermen Reaktion wird im allgemeinen noch bei Raumtemperatur oder gegebenenfalls mäßig erhöhter Temperatur innerhalb der genannten Temperaturbereiche nachgerührt, bis die dünnschichtchromatographische oder gaschromatographische Analyse vollständigen Umsatz anzeigt.

Die Aufarbeitung der Dinitroverbindungen erfolgt in an sich bekannter Weise. Die Dinitroverbindungen stellen oftmals in dem verwendeten Lösungsmittel schwer lösliche Substanzen darf, so daß die Hauptmenge beim Abkühlen des Reaktionsgemischs auf Raumtemperatur ausfällt und durch einfaches Filtrieren isoliert werden kann. Eine andere Art der Aufarbeitung besteht darin, das Reaktionsgemisch in Wasser einzurühren und das ausgefallene Reaktionsprodukt in üblicher Weise durch Filtration zu gewinnen. Im Falle öliger Reaktionsprodukte ist demgegenüber eine extraktive Aufarbeitung nach üblichen Methoden zweckmäßig, wobei als Extraktionsmittel für das mit Wasser vermischte Reaktionsgemisch beispielsweise Toluol, Methylenchlorid, Chlorbenzol, Dichlorbenzol, 1,2-Dichlorethan, Trichlorethylen u.a. in Betracht kommen.

Grundsätzlich denkbar, jedoch weniger bevorzugt, wäre auch eine Arbeitsweise, die darin besteht, das in der Stufe a) anfallende Reaktionsgemisch, gegebenenfalls nach Neutralisation des überschüssigen Alkalihydroxids, ohne Zwischenisolierung direkt der Stufe b) zuzuführen.

Die in der Stufe a) des erfindungsgemäßen Verfahrens erhaltenen Dinitroverbindungen werden in der Stufe b) in an sich bekannter Weise durch Reduktion mit nascierendem oder katalytisch, beispielsweise mittels Raney-Nickel oder Palladium auf Kohle angeregtem Wasserstoff in die entsprechenden Diamine überführt. Die Hydrierung erfolgt im allgemeinen in Gegenwart eines inerten Lösungsmittels der obengenannten Art - bevorzugt ist Dimethylformamid- oder in einem anderen Lösungsmittel wie z.B. Methanol, Ethanol oder Isopropanol bei 20 bis 120°C und einem Druck von 20 bis 80 bar. Die "Lösungsmittel" stellen hierbei für die Dinitroverbindungen zunächst Suspendiermittel dar, in denen sich die Diamine im Anschluß an ihre Bildung lösen, Die Diamine werden als Destillationsrückstand bei der destillativen Entfernung des Lösungsmittels gewonnen.

Die in der Stufe b) des erfindungsgemäßen Verfahrens anfallenden Diamine werden anschließend in der Stufe c) des erfindungsgemäßen Verfahrens mit Phosgen zu den entsprechenden Diisocyanaten umgesetzt. Hierbei kann man sowohl die freien Amine als auch deren Addukte mit Chlorwasserstoff oder Kohlendioxid einsetzen. Die Phosgenierung wird im allgemeinen in Chlorbenzol oder Dichlorbenzol durchgeführt, einem Reaktionsmedium, welches für die freien Amine in Abhängigkeit von ihrer Konstitution ein Suspendier- oder Lösungsmittel und für die Diisocyanate im allgemeinen ein Lösungsmittel darstellt. Die erfindungsgemäße Phosgenierung erfolgt im übrigen nach an sich bekannten Methoden wie sie beispielsweise in der DE-OS 3 442 689, in Liebigs Annalen der Chemie, Band 562, Jahrgang 1949, Seiten 75 bis 109, in Ullmanns Encyclopädie der technischen Chemie, Band 14, 4. Auflage, 1977, Seiten 350 bis 354 oder in Houben-Weyl, Methoden der organischen Chemie, Band E 4, 4. Auflage, 1983, Seiten 741 bis 753 beschrieben sind.

Das erfindungsgemäße Verfahren gestattet erstmals die Herstellung der neuen erfindungsgemäßen Diisocyanate, in denen die Alkylenbrücke verzweigt ist. Bei der Verwendung dieser erfindungsgemäßen Diisocyanate zur Herstellung von Polyurethankunststoffen, insbesondere von massiven oder zellförmigen Polyurethanelastomeren werden die erfindungsgemäßen Diisocyanate anstelle der bislang für diesen Verwendungszweck eingesetzten Diisocyanate mit den bekannten Reaktionspartnern zur Umsetzung gebracht (vgl. diesbezüglich beispielsweise die bereits eingangs genannten Literaturstellen oder auch "Kunststoff-Handbuch", Band VII, "Polyurethane" von Vieweg und Höchtlen, Carl Hanser Verlag

München (1966), insbesondere Seiten 206-297).

So erfolgt beispielsweise die Herstellung von Polyurethanelastomeren unter Verwendung des erfindungsgemäßen Diisocyanats durch dessen Umsetzung mit

a) Di- oder trifunktionellen Polyhydroxylverbindungen des Molekulargewichtsbereichs 400 bis 10000, vorzugsweise 800 bis 3000, vorzugsweise den entsprechenden Polyhydroxypolyestern oder Polyhydroxypolyethern,

b) Kettenverlängerungsmitteln des Molekulargewichtsbereichs 60 bis 399, d.h. mit im Sinne der Isocyanat-Additionsreaktion difunktionellen Verbindungen mit alkoholischen Hydroxylgruppen oder primären bzw. sekundären Aminogruppen, gegebenenfalls in Gegenwart von

c) weiteren aus der Chemie der Polyurethanelastomeren an sich bekannten Hilfs- und Zusatzmitteln.

Die Umsetzung kann nach dem bekannten Prepolymerverfahren (Umsetzung des Diisocyanats mit der Komponente a) unter Einhaltung eines Äquivalentverhältnisses von Isocyanatgruppen zu gegenüber Isocyanatgruppen reaktionsfähigen Gruppen von größer als 1,3:1 und anschließende Umsetzung des so erhaltenen NCO-Prepolymeren mit der Komponente b) oder aber auch einstufig durch Umsetzung des Diisocyanats mit einem Gemisch der Komponenten a) und b) erfolgen. Bei beiden Varianten liegt das Äquivalentverhältnis von Isocyanatgruppen zur Gesamtmenge der gegenüber Isocyanatgruppen reaktionsfähigen Gruppen im allgemeinen bei 0,8:1 bis 1,3:1, vorzugsweise 0,95:1 bis 1,1:1, Die Temperaturen, bei welchen diese Umsetzungen durchgeführt werden, liegen im allgemeinen bei 60 bis 180°C, vorzugsweise bei 80 bis 150°C. Die Umsetzungen können in Anwesenheit oder auch in Abwesenheit von geeigneten inerten Lösungsmitteln erfolgen.

Bei den mit den erfindungsgemäßen Diisocyanaten hergestellten Polyurethankunststoffen, insbesondere Polyurethanelastomeren kann es sich sowohl um massive als auch um zellförmige Produkte handeln. Die Herstellung beider Typen von Polyurethanelastomeren erfolgt nach den bekannten Verfahren, wie sie beispielsweise in der zuletzt genannten Literaturstelle beschrieben sind. So erfolgt beispielsweise die Herstellung von zellförmigen Polyurethanelastomeren unter Verwendung bzw. Mitverwendung von Wasser als Kettenverlängerungsmittel.

Die mit den erfindungsgemäßen Diisocyanaten hergestellten Kunststoffee insbesondere Elastomeren, besitzen teilweise hochwertige mechanische und thermische Eigenschaften. Sie sind deshalb hervorragend geeignet für Feder- und Dämpfungselemente, Puffer, Radbeläge, Dichtungen, Schuhsohlen und ähnliche Einsatzgebiete, bei denen das Material extremen mechanischen und thermischen Beanspruchungen ausgesetzt ist.

Im übrigen kann darauf verwiesen werden, daß die in der Stufe b) des erfindungsgemäßen Verfahrens als Zwischenprodukte anfallenden Diamine nicht nur als Vorprodukte für die Diisocyanate geeignet sind sondern ausgezeichnete Vernetzer oder Kettenverlängerungsmittel für Kunststoffvorläufer, beispielsweise für NCO-Prepolymere bei der Herstellung von Polyurethanelastomeren oder für Epoxidharze darstellen, wobei die Diamine anstelle der bislang für derartige Einsatzgebiete eingesetzten Polyamine verwendet werden.

Die nachfolgenden Beispiele dienen zur weiteren Erläuterung der Erfindung. Alle Prozentangaben beziehen sich auf Gewichtsprozente. Die mittlere Korngröße des eingesetzten gepulverten Natriumhydroxids liegt bei 6 bis 9 µm.

Vergleichsbeispiel

(Umsetzung von 2,2-Dimethyl-1,3-dihydroxypropan (Neopentylglykol) mit 4-Nitrochlorbenzol in Analogie zu DE-OS 2 634 419)

Eine Mischung aus 26 g (0,25 Mol) Neopentylglykol, 86,6 g (0,55 Mol) 4-Nitrochlorbenzol, 60 g 50%ige Natronlauge, 200 ml Chlorbenzol und 5,6 g Triethylbenzylammoniumchlorid wird 12 Stunden bei 90°C gerührt. Durch gaschromatographische Analyse wurde folgende Produktverteilung in der organischen Phase (Lösung in Chlorbenzol) festgestellt:

ca. 56% 4, 4'-Dichlorazoxybenzol
ca. 33% 2, 2-Dimethyl-3-(4-nitrophenoxy)-propanol
ca. 10% 4-Nitrochlorbenzol
ca. 1% 4-Nitrophenol

Bis-(4-nitrophenoxy)-2, 2-dimethylpropan kann allenfalls in Spuren nachgewiesen werden,

In den nachfolgenden Beispielen 1 bis 10 wird die Durchführung der Stufe a) des erfindungsgemäßen Verfahrens anhand einiger ausgewählter Reaktionspartner gezeigt.

Beispiel 1: (1,3-Bis-(4-nitrophenoxy)-2,2-dimethylpropan)

Zu einer Lösung von 104 g (1 Mol) Neopentylglykol und 346, 5 g (2, 2 Mol) 4-Nitrochlorbenzol in 600 ml Dimethylsulfoxid (DMSO) gibt man unter gutem Rühren über 3 Stunden 140 g gepulvertes Natriumhydroxid in kleinen Portionen zu, wobei während der Zugabe und anschließend bis zum Abklingen der exothermen Reaktion durch Eiskühlung eine Innentemperatur von 40 – 50°C eingehalten wird.

Anschließend rührt man noch 8 Stunden bei 25°C. Das ausgefallene Produkt wird abgesaugt, erst mit Wasser neutral, dann einmal mit Essigester gewaschen und anschließend im Vakuum bei 90°C getrocknet.

Ausbeute: 321 g (92,8% der Theorie) Fp.: 165 – 167°C (schwach gelbes Pulver)

Beispiel 2: (1,3-Bis-(4-nitrophenoxy)-2,2-dimethylpropan)

Gleicher Ansatz wie in Beispiel 1, jedoch Umsetzung in 600 ml Dimethylformamid (DMF) als Lösungsmittel.

Ausbeute: 242 g (69,9% der Theorie) Fp.: 165 – 167°C (beiges Pulver)

Beispiel 3: (1,3-Bis-(4-nitrophenoxy)-2,2-diethylpropan)

132 g (1 Mol) 2,2-Diethyl-1,3-dihydroxypropan, 346,5 g (2,2 Mol) 4-Nitrochlorbenzol und 140 g gepulvertes Natriumhydroxid werden analog Beispiel 1 in 600 ml DMSO umgesetzt.

Ausbeute: 277 g (74,1% der Theorie) Fp.: 108 – 109°C (beiges Pulver)

Beispiel 4: (1,3-Bis-(4-nitrophenoxy)-2-methyl-2-propylpropan)

132 g (1 Mol) 2-Methyl-2-propyl-1,3-dihydroxypropan, 346,5 g (2,2 Mol) 4-Nitrochlorbenzol und 140 g gepulvertes Natriumhydroxid werden analog Beispiel 1 in 600 ml DMSO umgesetzt.

Ausbeute: 310 g (82,9% der Theorie) Fp.: 124 – 125°C (beiges Pulver)

Beispiel 5: (1,3-Bis-(2-nitrophenoxy)-2,2-dimethylpropan)

104 g (1 Mol) Neopentylglykol, 346,5 g (2,2 Mol) 2-Nitrochlorbenzol und 140 g gepulvertes Natriumhydroxid werden analog Beispiel 1 in 600 ml DMSO umgesetzt.

Ausbeute: 203 g (58,7 % der Theorie) Fp.: 89 – 90°C (beiges Pulver)

In den Beispielen 6–9 wird die Hydrierung einiger der in den Beispielen 1–5 beschriebenen Bisnitrophenoxyalkane zu den entsprechenden, als Zwischenprodukte dienenden, Bisaminophenoxyalkanen erläutert (Stufe b) des erfindungsgemäßen Verfahrens):

Beispiel 6: (1, 3-Bis-(2-aminophenoxy)-2, 2-dimethylpropan)

343 g 1, 3-Bis-(2-nitrophenoxy)-2,2-dimethylpropan werden in 1800 ml DMF in Gegenwart von 53 g Raney-Nickel bei 60°C und 50 bar hydriert. Nach beendeter Wasserstoffaufnahme wird vom Katalysator abfiltriert und das Lösungsmittel im Vakuum abgezogen.

Ausbeute: 283 g (quantitativ) (dunkles Öl) (GC: 98, 7 %ig)

Beispiel 7: (1,3-Bis-(4-aminophenoxy)-2,2-dimethylpropan)

Analog Beispiel 10 werden 1949 g 1,3-Bis-(4-nitrophenoxy)-2,2-dimethylpropan in 10 1 DMF in Gegenwart von 300 g Raney-Nickel hydriert, Das Rohprodukt wird aus Ethanol/Isopropanol (1:1) umkristallisiert.

Ausbeute: 1466 g (91% der Theorie) Fp.: 116 – 117°C (farblose Kristalle)

Beispiel 8: (1,3-Bis-(4-aminophenoxy)-2,2-diethylpropan)

Analog Beispiel 10 werden 27 g 1,3-Bis-(4-nitrophenoxy)-2, 2-diethylpropan in 1400 ml DMF in Gegenwart von 42 g Raney-Nickel hydriert. Das Rohprodukt wird aus Isopropanol umkristallisiert.

Ausbeute: 160 g (70,6% der Theorie) Fp.: 100°C (farblose Kristalle)

Beispiel 9: (1, 3-Bis-(4-aminophenoxy)-2-methyl-2-propylpropan)

Analog Beispiel 10 werden 300 g 1, 3-Bis-(4-nitrophenoxy)-2-methyl-2-propylpropan in 1500 ml DMF in Gegenwart von 46 g Raney-Nickel hydriert. Das Rohprodukt wird aus Toluol umkristallisiert.

Ausbeute: 203 g (80,6% der Theorie) Fp.: 54 – 55°C (farblose Kristalle)

In den Beispielen 10–13 wird die Herstellung der erfindungsgemäßen Diisocyanate durch Phosgenierung der in den Beispielen 6–9 beschriebenen Bisaminophenoxyalkane erläutert (Stufe c) des erfindungsgemäßen Verfahrens):

Beispiel 10: (1,3-Bis-(2-isocyanatophenoxy)-2,2-dimethylpropan)

In einer 4 l-Laborphosgenierungsapparatur werden 2,5 l Chlorbenzol vorgelegt und anschließend ca. 400 g Phosgen einkondensiert. Zu dieser Lösung tropft man bei -10–0°C unter Rühren und langsamen Einleiten von Phosgen (40 bis 50 g/h) eine Lösung von 300 g 1,3-Bis-(2-aminophenoxy)2,2-dimethylpropan in 300 ml Chlorbenzol innerhalb einer Stunde zu. Nach Zugabe wird das Gemisch unter weiterem Phosgeneinleiten im Verlauf von 3 Stunden bis zum Rückfluß erhitzt und anschließend eine weitere Stunde am Rückfluß gekocht, wobei eine fast klare Lösung entsteht, Überschüssiges Phosgen wird durch Ausblasen mit Stickstoff entfernt und das ungelöste Material abfiltriert. Nach Abziehen des Lösungsmittels destilliert man den Rückstand im Vakuum. Dabei erstarrt das Produkt in der Vorlage.
Ausbeute: 241 g (71,3 % der Theorie) Kp.: 190 – 203°C/0, 4 mbar; Fp: 61 - 63°C NCO: ber.: 24,85%, gef.: 24,4% hydrolysierbares Chlor: 0, 007 %.

Beispiel 11: (1,3-Bis-(4-isocyanatophenoxy)-2,2-dimethylpropan)

In eine Suspension von 143 g 1,3-Bis-(4-aminophenoxy)2,2-dimethylpropan in 1400 ml Chlorbenzol leitet man unter Rühren bei -10 – 0°C zügig ca. 200 g Phosgen ein.
Die Phosgenierung wird, wie in Beispiel 10 beschrieben, zu Ende geführt.
Ausbeute: 160 g (94,7% der Theorie) Kp.: 185 – 200°C/0,1 mbar; Fp.: 50°C NCO: ber.: 24,85%, gef.: 24,8% hydrolysierbares Chlor: 0,005%

Beispiel 12: (1,3-Bis-(4-isocyanatophenoxy)-2,2-diethylpropan)

In eine Suspension von 157 g (1,3-Bis-(4-aminophenoxy)2,2-diethylpropan in 1500 ml Chlorbenzol leitet man unter Rühren bei –10 – 0°C zügig ca. 200 g Phosgen ein.
Die Phosgenierung wird, wie in Beispiel 10 beschrieben, zu Ende geführt.
Ausbeute: 153 g (83,6% der Theorie) Kp.: 215 – 225°C/0,2 mbar; Fp.: 56 – 57°C NCO: ber.: 23,0%, gef.: 23,0% hydrolysierbares Chlor: 0,002%

Beispiel 13: (1,3-Bis-(4-isocyanatophenoxy)-2-methyl-2-propylpropan)

210 g 1,3-Bis-(4-aminophenoxy)-2-methyl-2-propylpropan werden entsprechend den Angaben in Beispiel 10 in 1200 ml Chlorbenzol phosgeniert.
Ausbeute: 177 g (72,3% der Theorie) Kp.: 215 – 225°C/0,2 mbar (schwach gelbes Öl) Viskosität: 705 mPas/23°C, D (23°C): 1,036 NCO: ber.: 23,0%; gef.: 23,2% hydrolysierbares Chlor: 0,027%

**Patentansprüche**

1. Diisocyanate der allgemeinen Formel

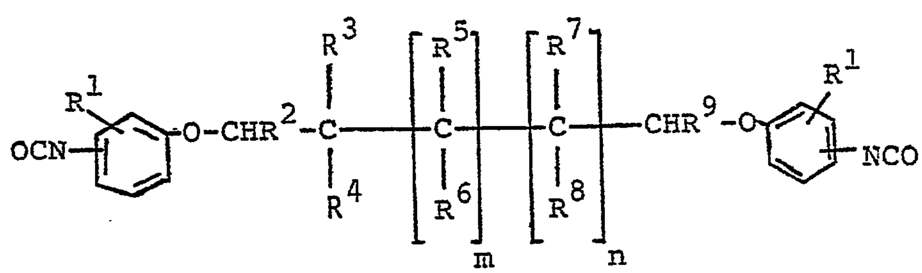

für welche $R^1$ für Wasserstoff oder eine Methylgruppe steht und $R^2$, $R^3$, $R^5$, $R^6$, $R^7$, $R^8$ und $R^9$ für Wasserstoff oder Alkylreste mit 1 bis 4 Kohlenstoffatomen stehen, $R^4$ für einen Alkylrest mit 1 bis 4 Kohlenstoffatomen steht und m und n jeweils für 0 oder 1 stehen.

2. Diisocyanate gemäß Anspruch 1 für welche $R^1$ für Wasserstoff steht, $R^2$, R5, $R^6$, $R^7$ und $R^9$ für Wasserstoff stehen: $R^3$ und $R^4$ für Methylgruppen stehen, R8 für Wasserstoff oder eine Methylgruppe steht und m und n die in Anspruch 1 genannte Bedeutung haben, und in welchen die Diisocyanatgruppen ortho- oder paraständig zu dem mit dem aromatischen Ring verknüpften Sauerstoffatom angeordnet sind.

3. Verfahren zur Herstellung von Diisocyanaten gemäß Anspruch 1 durch

a) Umsetzung von (i) ein- oder zweiwertigen Alkoholen der Formeln

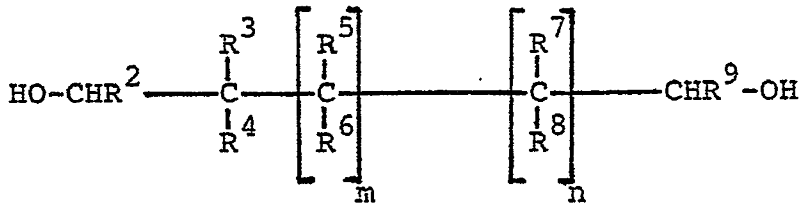

oder

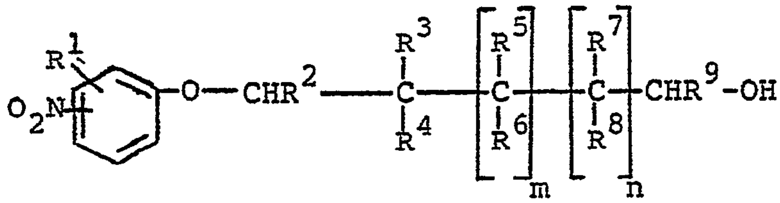

mit (ii) Verbindungen der allgemeinen Formel

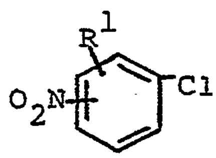

zu den entsprechenden Dinitroverbindungen
wobei in diesen Formeln $R^1$ für Wasserstoff oder eine Methylgruppe steht,
$R^2$, $R^3$, $R^5$, $R^6$, $R^7$, $R^8$ und $R^9$ für Wasserstoff oder Alkylreste mit 1 bis 4 Kohlenstoffatomen stehen,
$R^4$ für einen Alkylrest mit 1 bis 4 Kohlenstoffatomen steht und m und n jeweils für 0 oder 1 stehen,
b) an sich bekannte Hydrierung der zuletzt genannten Dinitroverbindungen zu den entsprechenden diprimären Diaminen und c) an sich bekannte Phosgenierung der gemäß b) erhaltenen Diaminoverbindungen,
dadurch gekennzeichnet, daß man die Umsetzung a) in Gegenwart von gepulvertem Natrium- und/oder Kaliumhydroxid in einer zur Neutralisation des abgespalteten Chlorwasserstoffs mindestens ausreichenden Menge und in Gegenwart eines stark polaren, aprotischen Lösungsmittels durchführt.
4. Verfahren zur Herstellung von Diisocyanaten gemäß Anspruch 2 durch
a) Umsetzung von (i) ein- oder zweiwertigen Alkoholen der Formeln

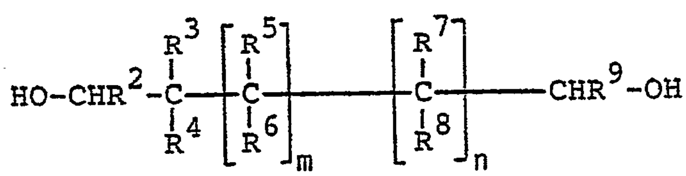

oder

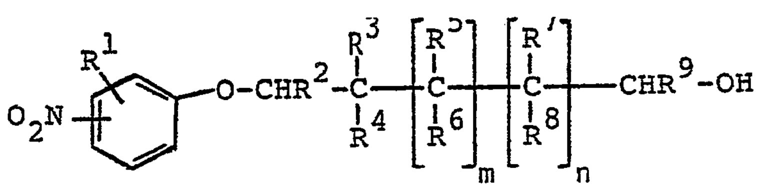

mit (ii) Verbindungen der allgemeinen Formel

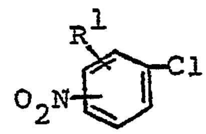

zu den entsprechenden Dinitroverbindungen wobei in diesen Formeln $R^1$ für Wasserstoff steht,
$R^2$, $R^5$, $R^6$, $R^7$ und $R^9$ für Wasserstoff stehen,
$R^3$ und $R^4$ für Methylgruppen stehen,
$R^8$ für Wasserstoff oder eine Methylgruppe steht und m und n die in Anspruch 3 genannte Bedeutung haben,
b) an sich bekannte Hydrierung der zuletzt genannten Dinitroverbindungen zu den entsprechenden diprimären Diaminen und
c) an sich bekannte Phosgenierung der gemäß b) erhaltenen Diaminoverbindungen, dadurch gekennzeichnet, daß man die Umsetzung a) in Gegenwart von gepulvertem Natrium- und/oder Kaliumhydroxid in einer zur Neutralisation des abgespalteten Chlorwasserstoffs mindestens ausreichenden Menge und in Gegenwart eines stark polaren, aprotischen Lösungsmittels durchführt.

5. Verwendung der Diisocyanate gemäß Anspruch 1 und 2 als Aufbaukomponente bei der Herstellung von Polyurethankunststoffen nach dem Isocyanat-Polyadditionsverfahren.

**Claims**

1. Diisocyanates corresponding to the following general formula

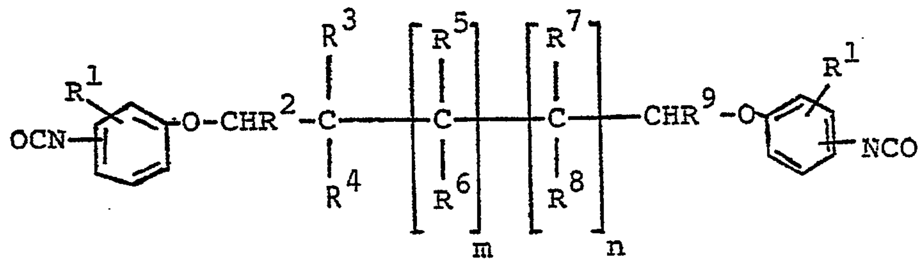

in which
$R^1$ stands for hydrogen or a methyl group and
$R^2$, $R^3$, $R^5$, $R^6$, $R^7$, $R^8$ and $R^9$ stand for hydrogen or alkyl groups having 1 to 4 carbon atoms,
$R^4$ stands for an alkyl group having 1 to 4 carbon atoms and
m and n each stands for 0 or 1.

2. Diisocyanates according to Claim 1 in which
$R^1$ stands for hydrogen,
$R^2$, $R^5$, $R^6$, $R^7$ and $R^9$ stand for hydrogen,
$R^3$ and $R^4$ stand for methyl groups,
$R^8$ stands for hydrogen or a methyl group and
m and n have the meanings indicated in Claim 1, and in which the diisocyanate groups are in the ortho- or para-position to the oxygen atom attached to the aromatic ring.

3. A process for the preparation of diisocyanates according to Claim 1 by
a) the reaction of (i) monohydric or dihydric alconhols corresponding to the following formula

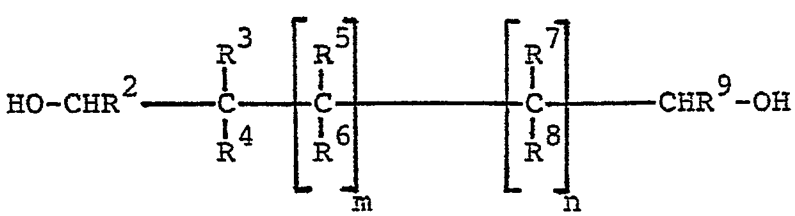

or

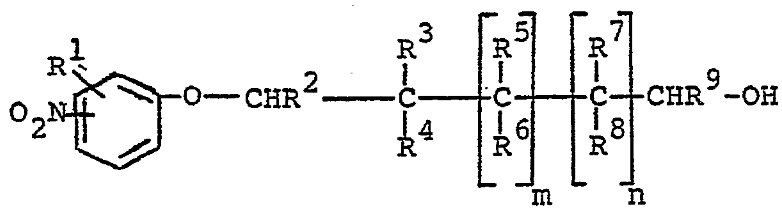

with (ii) compounds corresponding to the following general formula

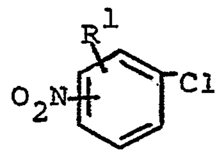

to form the corresponding dinitro compounds, in which formulae, $R^1$ stands for hydrogen or a methyl group,
$R^2$, $R^3$, $R^5$, $R_6$, $R^7$, $R^8$ and $R^9$ stand for hydrogen or alkyl groups having 1 to 4 carbon atoms,
$R^4$ stand for an alkyl group having 1 to 4 carbon atoms and
m and n each stands for 0 or 1,
b) hydrogenation in known manner of the last mentioned dinitro compounds to the corresponding diprimary diamines and
c) phosgenation in known manner of the diamino compounds obtained according to b),
characterised in that reaction a) is carried out in the
presence of pulverulent sodium hydroxide and/or potassium hydroxide in a quantity at least sufficient for neutralisation of the hydrogen chloride split off and in the presence of a highly polar, aprotic solvent.
4. A process for the preparation of diisocyanates according to Claim 2, by
a) the reaction of
(i) monohydric or dihydric alcohols corresponding to the following formulae

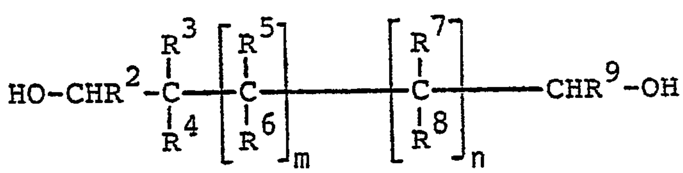

or

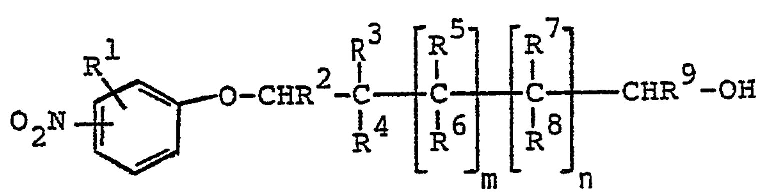

with (ii) compounds corresponding to the following general formula

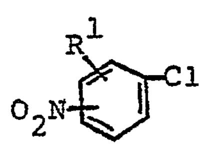

to form the corresponding dinitro compounds, in which formulae, $R^1$ stands for hydrogen,
$R^2$, $R^5$, $R^6$, $R^7$ and $R^9$ stand for hydrogen,
$R^3$ and $R^4$ stand for methyl groups,
$R^8$ stands for hydrogen or a methyl group and
m and n have the meanings indicated in Claim 3,

b) hydrogenation in known manner of the last-mentioned dinitro compounds to the corresponding diprimary diamines and

c) phosgenation in known manner of the diamino compounds obtained according to b),

characterised in that reaction a) is carried out in the presence of pulverulent sodium hydroxide and/or potassium hydroxide in a quantity at least sufficient for neutralisation of the hydrogen chloride split off and in the presence of a highly polar, aprotic solvent.

5. Use of the diisocyanates according to claims 1 and 2 as synthesis components for the production of polyurethane resins by the isocyanate polyaddition process.

## Revendications

1. Diisocyanates de formule générale

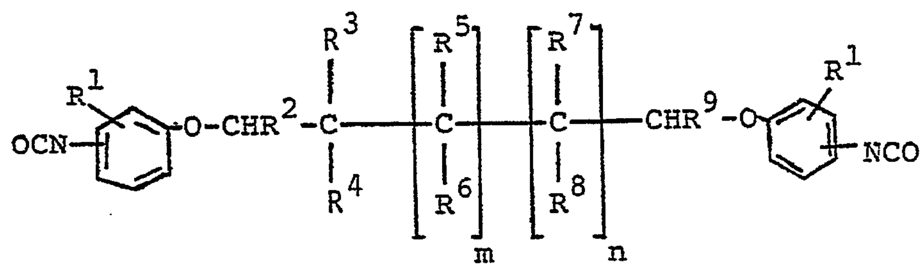

dans laquelle
$R^1$ représente l'hydrogène ou un groupe méthyle et
$R^2$, $R^3$, $R^5$, $R^6$, $R^7$, $R^8$ et $R^9$ représentent de l'hydrogène ou des restes alkyle ayant 1 à 4 atomes de carbone,
$R^4$ est un reste alkyle ayant 1 à 4 atomes de carbone et
m et n ont chacun la valeur 0 ou 1.

2. Diisocyanates suivant la revendication 1, dans lesquels
$R^1$ désigne l'hydrogène,
$R^2$, $R^5$, $R^6$, $R^7$ et $R^9$ représentent de l'hydrogène
$R^3$ et $R^4$ sont des groupes méthyle
$R^8$ est l'hydrogène ou un groupe méthyle et
m et n ont la définition mentionnée dans la revendication 1,
et dans lesquels les groupes diisocyanate sont en ortho ou para par rapport à l'atome d'oxygène lié au noyau aromatique.

3. Procédé de préparation de diisocyanates suivant la revendication 1, par

a) réaction (i) d'alcools monovalents ou divalents de formules

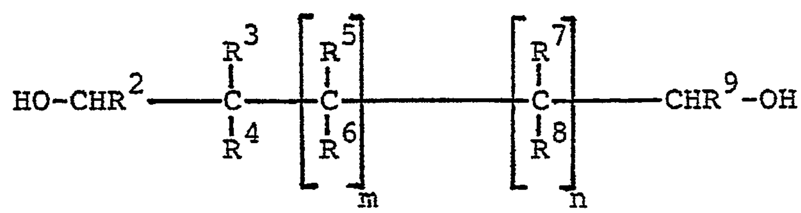

ou

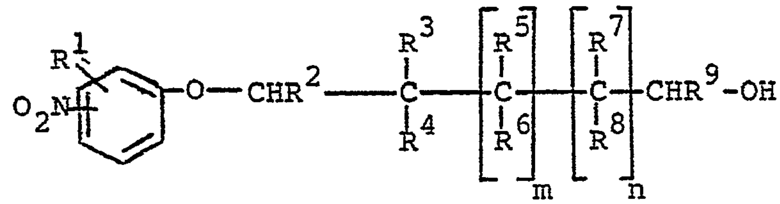

avec (ii) des composés de formule générale

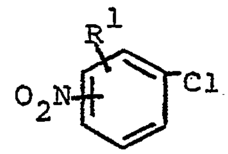

pour former les composés dinitrés correspondants, formules dans lesquelles
$R^1$ désigne l'hydrogène ou un groupe méthyle
$R^2$, $R^3$, $R^5$, $R^6$, $R^7$, $R^8$ et $R^9$ représentent de l'hydrogène ou des restes alkyle ayant 1 à 4 atomes de carbone,
$R^4$ désigne un reste alkyle ayant 1 à 4 atomes de carbone et
m et n ont chacun la valeur 0 ou 1,
b) hydrogénation connue des composés dinitrés mentionnés en dernier lieu en les diamines diprimaires correspondantes et
c) phosgénation connue des composés diaminés obtenus conformément à b),
caractérisé en ce qu'on conduit la réaction a) en présence d'hydroxyde de sodium et/ou d'hydroxyde de potassium en poudre en une quantité au moins suffisante pour la neutralisation du chlorure d'hydrogène libéré et en présence d'un solvant aprotique fortement polaire.

4. Procédé de préparation de diisocyanates suivant la revendication 2, par
a) réaction (i) d'alcools monovalents ou divalents de formules

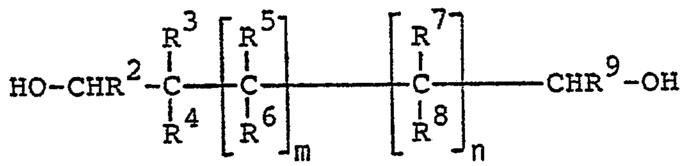

ou

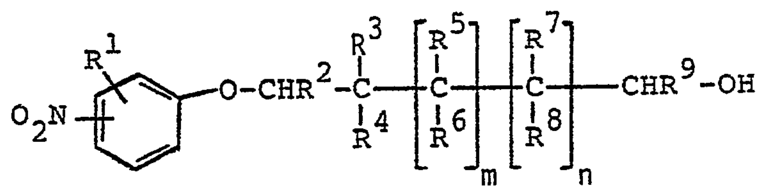

avec (ii) des composés de formule générale

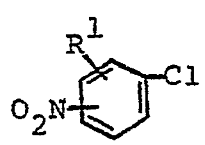

pour former les composés dinitrés correspondants, formules dans lesquelles
$R^1$ désigne l'hydrogène,
$R^2$, $R^5$, $R^6$, $R^7$ et $R^9$ sont de l'hydrogène,
$R^3$ et $R^4$ représentent les groupes méthyle,
$R^8$ est l'hydrogène ou un groupe méthyle et
m et n ont la définition mentionnée dans la revendication 3
b) hydrogénation connue des composés dinitrés mentionnés en dernier lieu en les diamines diprimaires correspondantes et
c) phosgénation connue des composés diaminés obtenus conformément à b),
caractérisé en ce qu'on conduit la réaction a) en présence d'hydroxyde de sodium et/ou d'hydroxyde de potassium en poudre en une quantité au moins suffisante pour la neutralisation du chlorure d'hydrogène libéré et en présence d'un solvant aprotique fortement polaire.

5. Utilisation des diisocyanates suivant les revendications 1 et 2 comme composants structuraux dans la production de matières plastiques du type polyuréthanne par le procédé de polyaddition d'isocyanate.